# EUROPÄISCHE PATENTSCHRIFT

(11) **EP 1 020 424 B1**
(45) Veröffentlichungstag und Bekanntmachung des Hinweises auf die Patenterteilung: **03.04.2002**
(21) Anmeldenummer: 99124994.7
(22) Anmeldetag: 15.12.1999
(51) Int. Cl.: C07C 209/26

(54) **Verfahren zur Herstellung von N-Ethyl-diisopropylamin**
Method for the production of N-Ethyl-diisopropylamine
Procédé pour le préparation de Amine N-Ethyl-diisopropyl

(30) Priorität: 14.01.1999 DE 19901135
(43) Veröffentlichungstag der Anmeldung: 19.07.2000
(73) Patentinhaber: BASF AKTIENGESELLSCHAFT, 67056 Ludwigshafen (DE)
(72) Erfinder: Eller, Karsten, Dr., 67061 Ludwigshafen (DE); Fiege, Bernd, 67227 Frankenthal (DE); Henne, Andreas, Dr., 67433 Neustadt (DE); Kneuper, Heinz-Josef, Dr., 67150 Niederkirchen (DE)

(56) Entgegenhaltungen:
- CHEMICAL ABSTRACTS, vol. 113, no. 19, 5. November 1990 (1990-11-05) Columbus, Ohio, US; abstract no. 171481, NAHATA, TOSHINARI: "Preparation of N,N-diisopropylethylamine" XP002134320 & JP 02 180854 A (KOEI CHEMICAL CO., LTD., JAPAN) 13. Juli 1990 (1990-07-13)

## Beschreibung

Die vorliegende Erfindung betrifft ein Verfahren zur Herstellung von N-Ethyl-diisopropylamin (Hünig-Base) der Formel 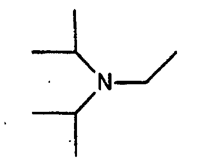 durch Umsetzung von Acetaldehyd mit Diisopropylamin und Wasserstoff bei erhöhter Temperatur und unter Druck in Gegenwart eines Hydrierkatalysators.

N-Ethyl-diisopropylamin ist ein wichtiges Amin, das als starke, wenig nukleophile Base in Eliminierungsreaktionen eingesetzt wird und als Katalysator bzw. Hilfsbase in der organischen Synthese von beispielsweise Wirkstoffen (siehe WO 98/07430) Verwendung findet.

In Chem. Ber. 91, 380-92 (1958) wird die Synthese von N-Ethyl-diisopropylamin durch Umsetzung von Diisopropylamin mit Diethylsulfat beschrieben. Hierbei fallen Sulfate als unerwünschte Nebenprodukte an, die anschließend aufwendig entsorgt werden müssen.

Auch die in J. Org. Chem. 16, 1911-20 (1951) und US-A-2,692,285 beschriebene Umsetzung des Diisopropylamins mit Ethyliodid zur Synthese von N-Ethyl-diisopropylamin führt zu unerwünschten Salzen als Nebenprodukten.

JP-A-02 180 854 offenbart ein diskontinuierliches Verfahren zur Herstellung von N-Ethyl-diisopropylamin durch reduktive Dialkylierung von Ethylamin mit Aceton in Gegenwart eines Edelmetallkatalysators. Nachteilig an diesem Verfahren sind die geringen Ausbeuten.

JP-A-02 180 854 offenbart weiterhin ein diskontinuierliches Verfahren zur Herstellung von N-Ethyl-diisopropylamin durch Umsetzung von Diisopropylamin mit Acetaldehyd und Wasserstoff in Gegenwart eines Edelmetallkatalysators, wie Pd/C, Ru/C, Rh/C und Pt/C, wobei die Umsetzung in einem Autoklaven derart ausgeführt wird, dass der Acetaldehyd während der Umsetzung der Reaktionsmischung allmählich zugeführt wird (vergl. loc. cit.: Anspruch 2 und Beispiele Nr. 5 und 6), um gute Ausbeuten zu erzielen.

Gemäß der Lehre von JP-A-02 180 854 sind die Ausbeuten an N-Ethyl-diisopropylamin bei der Umsetzung von Diisopropylamin mit Acetaldehyd und Wasserstoff schlecht (kleiner 10 %), wenn das Diisopropylamin und der Acetaldehyd von Beginn an zusammen im Autoklav vorgelegt werden (loc. cit.: Vergleichsbeispiel 1).

Nachteilig an dem Verfahren sind die geringen Raum-Zeit-Ausbeuten aufgrund der diskontinuierlichen Fahrweise.

Eine kontinuierliche Fahrweise zur Herstellung von N-Ethyl-diisopropylamin würde gemäß der Lehre von JP-A-02 180 854 aufwendigerweise die Durchführung der katalytischen Umsetzung von Acetaldehyd mit Diisopropylamin und Wasserstoff in einer Reaktorkaskade, z. B. Rührkesselkaskade, wobei jedem Reaktor der Kaskade ein Teil des umzusetzenden Acetaldehyds zugeführt wird, oder einem Rohrreaktor mit mehreren Zulaufstellen für den Acetaldehyd entlang des Reaktors, z. B. einem Kompartmentreaktor mit mehreren Zulaufstellen, erfordern, wie dies z. B. in O. Levenspiel, 'Chemical Reaction Engineering', 2^{nd} Ed., Seiten 164-168, insbesondere 166-167, John Wiley (1972) allgemein für ähnlich gelagerte Fälle gelehrt wird.

Der vorliegenden Erfindung lag daher die Aufgabe zugrunde, ein verbessertes, unaufwendiges, wirtschaftliches, diskontinuierlich und vor allem kontinuierlich ausführbares Verfahren zur Herstellung von N-Ethyl-diisopropylamin mit guten Raum-Zeit-Ausbeuten aufzufinden.

Demgemäß wurde ein Verfahren zur Herstellung von N-Ethyl-diisopropylamin durch Umsetzung von Acetaldehyd mit Diisopropylamin und Wasserstoff bei erhöhter Temperatur und unter Druck in Gegenwart eines Hydrierkatalysators gefunden, welches dadurch gekennzeichnet ist, dass der Katalysator ein oxidisches Trägermaterial, ausgewählt aus der Gruppe Zirkoniumdioxid, Titandioxid, Aluminiumoxid, Siliziumdioxid, Zinkoxid, Magnesiumoxid, Cerdioxid, Tone und Zeolithe oder Mischungen daraus, enthält.

Im allgemeinen werden im erfindungsgemäßen Verfahren die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch inaktiven Begleitstoffe enthalten.

In diesem Zusammenhang werden die oxidischen Trägermaterialien Zirkoniumdioxid (ZrO₂), Titandioxid (TiO₂), Aluminiumoxid (Al₂O₃), Siliziumdioxid (SiO₂), Zinkoxid (ZnO), Magnesiumoxid (MgO), Cerdioxid (CeO₂), Tone und Zeolithe als zur katalytisch aktiven Masse gehörig gewertet.

Die Katalysatoren werden dergestalt eingesetzt, dass man die katalytisch aktive, zu Pulver vermahlene Masse in das Reaktionsgefäss einbringt oder bevorzugt, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z.B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o.g. Katalysatorträgermaterialien definiert und enthält im wesentlichen die Bestandteile
Zirkoniumdioxid (ZrO₂), Titandioxid (TiO₂), Aluminiumoxid (Al₂O₃), Siliziumdioxid (SiO₂), Zinkoxid (ZnO), Magnesiumoxid (MgO), Cerdioxid (CeO₂), Tone, Zeolithe oder Gemische zweier oder mehrerer dieser Komponenten und ein oder mehrere Metalle oder deren Oxide, ausgewählt aus der Gruppe Cr, Mo, W, Re, Ru, Rh, Pd, Os, Ir, Pt, Ag, Au, Fe, Co, Ni und Cu.

Die Summe der o.g. wesentlichen Bestandteile der katalytisch aktiven Masse - wobei die Komponenten Cr, Mo, W, Re, Ru, Rh, Pd, Os, Ir, Pt, Ag, Au, Fe, Co, Ni und/oder Cu als Metall in der Oxidationsstufe 0 berechnet werden - beträgt üblicherweise 70 bis 100 Gew.-%, bevorzugt 80 bis 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A, II A, III A, IV A, V A, VI A, I B, II B, III B, IV B und V B des Periodensystems, enthalten.

Beispiele für solche Elemente bzw. deren Verbindungen sind:

Übergangsmetalle, wie Mn bzw. Mn₂O₃; Lanthanide, wie Pr bzw. Pr₂O₃; Alkalimetalloxide, wie Na₂O; Alkalimetallcarbonate; Erdalkalimetalloxide, wie CaO; Erdalkalimetallcarbonate, wie CaCO₃; Boroxid (B₂O₃); Nioboxalat; Vanadylpyrophosphat.

Die katalytisch aktive Masse der im erfindungsgemäßen Verfahren eingesetzten Katalysatoren enthält im allgemeinen nach deren letzter Wärmebehandlung und vor dessen Reduktion mit Wasserstoff 50 bis 99,9 Gew.-%, bevorzugt 60 bis 99,9 Gew.-%, besonders bevorzugt 80 bis 99,9 Gew.-%, ZrO₂ und/oder TiO₂ und/oder Al₂O₃ und/ oder SiO₂ und/oder ZnO und/oder MgO und/oder CeO₂ und/oder Tone und/oder Zeolithe,
0,1 bis 50 Gew.-%, bevorzugt 0,1 bis 40 Gew.-%, besonders bevorzugt 0,1 bis 20 Gew.-%, ganz besonders bevorzugt 0,1 bis 5 Gew.-%, Cr, Mo, W, Re, Ru, Rh, Pd, Os, Ir, Pt, Ag, Au, Fe, Co, Ni und/oder Cu, berechnet als Metall in der Oxidationsstufe 0, und 0 bis 30 Gew.-%, bevorzugt 0 bis 25 Gew.-%, besonders bevorzugt 0 bis 20 Gew.-%, ein oder mehrere Elemente (Oxidationsstufe 0) oder deren anorganische oder organische Verbindungen, ausgewählt aus den Gruppen I A, II A, III A, IV A, V A, VI A, I B, II B, III B, IV B und V B des Periodensystems.

Bevorzugte Katalysatoren enthalten in ihrer katalytisch aktiven Masse 50 bis 99,9 Gew.-% ZrO₂ und/oder TiO₂ und/oder Al₂O₃ und/ oder SiO₂ und/oder MgO, besonders bevorzugt 60 bis 99,9 Gew.-% ZrO₂ und/oder Al₂O₃ und/oder SiO₂ und/oder MgO, ganz besonders bevorzugt 60 bis 99,9 Gew.-% ZrO₂.

Bevorzugte Katalysatoren enthalten in ihrer katalytisch aktiven Masse 0,1 bis 20 Gew.-%, insbesondere 0,1 bis 5 Gew.-%, Re, Pd, Pt, Ru, Os, Rh, Ir, Ag und/oder Au, berechnet als Metall in der Oxidationsstufe 0.

Mehr bevorzugte Katalysatoren enthalten in ihrer katalytisch aktiven Masse 0,1 bis 5 Gew.-% Pd und/oder Pt und/oder Ag und/oder Ru, besonders bevorzugt 0,1 bis 5 Gew.-% Pd und/oder Pt, ganz besonders bevorzugt 0,1 bis 1,3 Gew.-% Pd und 0,1 bis 1,3 Gew.-% Pt, wobei die Edelmetallgehalte jeweils als Metall in der Oxidationsstufe 0 berechnet sind.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Katalysatorkomponenten mit Wasser und nachfolgendes Extrudieren und Tempern der so erhaltenen Masse erhältlich.

Zur Herstellung der im erfindungsgemäßen Verfahren verwendeten Katalysatoren können Fällungsmethoden angewendet werden. So können sie beispielsweise durch eine gemeinsame Fällung der MetallKomponenten aus einer diese Metalle enthaltenden, wässrigen Salzlösung mittels Mineralbasen in Gegenwart einer Aufschlämmung oder Suspension feinkörniger Pulver des schwerlöslichen Katalysatorträgermaterials, z. B. einer schwerlöslichen sauerstoffhaltigen Aluminium-, Titan-, Silizium- und/oder Zirkonium-Verbindung, und anschließendes Waschen, Trocknen und Calcinieren des erhaltenen Präzipitats erhalten werden. Als schwerlösliche, sauerstoffhaltige Aluminium-, Titan-, Silizium- und/oder Zirkonium-Verbindungen können beispielsweise Aluminiumoxid, Titandioxid, Zirkoniumdioxid, Zirkoniumoxidhydrat und Siliziumdioxid Verwendung finden, die vorteilhaft als Aufschlämmung durch Ausfällen der schwerlöslichen Aluminium-, Titan-, Silizium- und/oder Zirkonium-Verbindungen aus entsprechenden wässrigen Salzlösungen mittels Mineralbasen erhalten werden.

Vorteilhaft werden die im erfindungsgemäßen Verfahren verwendeten Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre, zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden wie üblich zu den erfindungsgemäßen Katalysatoren weiterverarbeitet. Nach dem Waschen werden sie im allgemeinen bei 80 bis 200 °C, vorzugsweise bei 100 bis 150 °C, getrocknet und danach calciniert. Die Calcinierung wird im allgemeinen bei Temperaturen zwischen 300 und 800 °C, vorzugsweise 400 bis 600 °C, insbesondere 450 bis 550 °C, ausgeführt.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z. B. Tabletten, verpresst und tempert. Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Bevorzugt werden die im erfindungsgemäßen Verfahren verwendeten Katalysatoren durch Tränkung von Zirkoniumdioxid (ZrO₂), Titandioxid (TiO₂), Aluminiumoxid (Al₂O₃), Siliziumdioxid (SiO₂), Zinkoxid (ZnO), Magnesiumoxid (MgO), Cerdioxid (CeO₂), Tonen oder Zeolithen oder Gemischen zweier oder mehrerer dieser Trägermaterialien, die beispielsweise in Form von Pulver oder Formkörpern, wie Strängen, Tabletten, Kugeln oder Ringen, vorliegen, hergestellt.

Aluminiumoxid kann in verschiedenen Modifikationen eingesetzt werden, bevorzugt sind α-, γ- oder θ-Al₂O₃.

Zirkoniumdioxid wird vorzugsweise in der monoklinen oder tetragonalen Form, ganz besonders bevorzugt in der monoklinen Form, und Titandioxid vorzugsweise als Anatas oder Rutil eingesetzt.

Siliciumdioxid kann z. B. über eine Fällung aus Wasserglas oder über das Sol-Gel-Verfahren erhalten und eingesetzt oder als mesoporöses SiO₂, z. B. als mesoporöses SiO₂ mit einer spezifischen Oberfläche der Mesoporen von mindestens 500 m²/g und einem Porenvolumen der Mesoporen von mindestens 1,0 ml/g gemäß DE-A-196 39 016, oder als Kieselgel (z. B. nach Ullmann, Enzykl. Techn. Chem., 4. Auflage, Band 21, S. 457-63, 1982) oder in Form von Silikaten, wie Alumosilikaten (z. B. gemäß Nature, Band 359, S. 710-12, 1992), Magnesiumsilikaten (z. B. Steatit), Zirkoniumsilikaten, Cersilikaten oder Calciumsilikaten, eingesetzt werden.

Geeignete Tone als Trägermaterialien bestehen überwiegend aus Phyllosilikaten und/oder Bandsilikaten. Gut geeignet sind beispielsweise Montmorillonit, Kaolin oder Hectorit.

Bei geeigneten zeolithischen Trägermaterialien handelt es sich um Alkali- bzw. Erdalkali-Alumosilikate, z. B. der allgemeinen Formel M_{2/z}O • Al₂O₃ • xSiO₂ • yH₂O, wobei M ein ein- oder mehrwertiges Metall, H, [NH₄], z die Wertigkeit, x = 1,8 bis ca. 12 und y = 0 bis ca. 8 bedeuten. Gut geeignet sind z. B. Faujasite oder Pentasile.

Die Herstellung von Formkörpern der o.g. oxidischen Trägermaterialien kann nach den üblichen Verfahren erfolgen.

Die Tränkung dieser oxidischen Trägermaterialien erfolgt ebenfalls nach den üblichen Verfahren, wie z. B. in EP-A-599 180, EP-A-673 918 oder A. B. Stiles, Catalyst Manufacture -Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und ggf. calciniert.

Die Tränkung kann nach der sogenannten "incipient wetness"-Methode erfolgen, bei der das oxidische Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft besonders dann anzuwenden, wenn das oxidische Trägermaterial mit einer größeren Metallmenge beaufschlagt werden soll.

Zur Aufbringung mehrerer Metallkomponenten auf das oxidische Trägermaterial kann die Tränkung gleichzeitig mit allen Metallsalzen oder in beliebiger Reihenfolge der einzelnen Metallsalze nacheinander erfolgen.

Der im erfindungsgemäßen Verfahren verwendete Katalysator kann vor seinem Einsatz reduziert werden. Die Reduktion kann drucklos oder unter Druck erfolgen. Wenn drucklos reduziert wird, geht man so vor, dass der Katalysator unter Inertgas, beispielsweise Stickstoff, bis zu der Reduktionstemperatur erhitzt wird und dann langsam das Inertgas gegen Wasserstoff ersetzt wird.

Bei einer Reduktion unter Druck geht man praktischerweise so vor, dass man bei den später auch in der Hydrierreaktion angewendeten Drücken und Temperaturen auch die Reduktion vornimmt. Je nach Temperatur und Wasserstoffdruck muss die Dauer der Reduktion gewählt werden, d. h. je drastischer die Bedingungen sind, desto kürzer kann die Reduktionszeit gewählt werden.

Im allgemeinen wird bei einer Temperatur von 80 bis 250 °C, einem Wasserstoffdruck von 0,5 bis 350 bar und einer Dauer von 1 bis 48 h reduziert.

Es ist jedoch ebenfalls möglich, den nicht reduzierten Katalysator im erfindungsgemäßen Verfahren einzusetzen. In diesem Fall erfolgt die Reduktion des Katalysators dann gleichzeitig unter den Verfahrensbedingungen. Nach einer kurzen Betriebszeit des erfindungsgemäßen Verfahrens von einigen Stunden oder wenigen Tagen ist die Reduktion des Katalysators üblicherweise nahezu vollständig.

Die Reduktion des Katalysators kann ganz oder teilweise separat von dem Aminierungsreaktor in einem geeigneten Apparat erfolgen oder direkt vor Beginn der Aminierung im Aminierungsreaktor.

Das erfindungsgemäße Verfahren lässt sich wie folgt ausführen:

Das erfindungsgemäße Verfahren lässt sich diskontinuierlich oder besonders bevorzugt kontinuierlich durchführen, wobei der Katalysator bevorzugt als Festbett im Reaktor angeordnet ist.

Das Verfahren kann in der Flüssigphase oder in der Gasphase durchgeführt werden. Bevorzugt ist das Festbettverfahren in der Flüssigphase. Je nach den gewählten Reaktionsbedingungen (Druck, Temperatur) wird jedoch immer ein gewisser Anteil der Edukte entsprechend dem Partialdruck gasförmig vorliegen.

Die beiden Edukte Diisopropylamin und Acetaldehyd können in einem stöchiometrischen, über- oder unterstöchiometrischen Molverhältnis eingesetzt werden. Das Molverhältnis Diisopropylamin zu Acetaldehyd liegt im allgemeinen bei 0,5 bis 10 mol/mol.

Wird das Verfahren in Gegenwart eines Lösungsmittels durchgeführt, beträgt das molare Verhältnis von Diisopropylamin zu Acetaldehyd im allgemeinen 0,6 bis 2, bevorzugt 0,7 bis 1,5, besonders bevorzugt 0,9 bis 1,2, ganz besonders bevorzugt 1,0 bis 1,2 mol/mol.

Wird das Verfahren in Abwesenheit eines Lösungsmittels durchgeführt, beträgt das molare Verhältnis von Diisopropylamin zu Acetaldehyd im allgemeinen 1 bis 5, bevorzugt 1,2 bis 4,0, besonders bevorzugt 1,3 bis 3,5 mol/mol.

Die beiden Edukte Diisopropylamin und Acetaldehyd werden in reiner, d. h. unverdünnter, Form oder als Lösung in einem inerten Lösungsmittel im erfindungsgemäßen Verfahren eingesetzt.

Bevorzugte Lösungsmittel sind Wasser, Alkohole, wie Methanol und Ethanol, Ether, wie THF, und Amide, wie DMAC, DMF und NMP. Besonders bevorzugte Lösungsmittel sind Wasser, Ethanol und NMP. Die Lösungen werden im allgemeinen in einer Konzentration von 5 bis 80 Gew.-%, bevorzugt 20 bis 70 Gew.-%, eingesetzt.

Der Wasserstoff wird der Reaktion im allgemeinen in einem großen molaren Überschuss bezogen auf den Acetaldehyd zugeführt. Üblicherweise wird mit der maximalen Leistung der Gasumlaufpumpe gearbeitet. Typische Bereiche sind 0,5 bis 15, bevorzugt 1 bis 5, Nm³ / l_{Kat.} / h (Nm³ = auf Normalbedingungen umgerechnetes Gasvolumen). Der tatsächliche Wasserstoffverbrauch wird über die Druckregelung nachgeführt.

Bei der bevorzugten kontinuierlichen Durchführung des Verfahrens in einem Festbettreaktor, z. B. Rohrreaktor, liegt die Belastung üblicherweise bei 0,03 bis 1,5 kg_{Acetaldehyd}l_{Kat.}⁻¹h⁻¹, bevorzugt bei 0,05 bis 1,0 kg_{Acetaldehyd}l_{Kat.}⁻¹h⁻¹, wobei das eventuell gegenwärtige Lösungsmittel für die Berechnung der Belastung nicht berücksichtigt wird. Die Angaben zur Katalysatorbelastung beziehen sich demnach auf den Acetaldehyd, berechnet 100 %, unabhängig davon, ob dieser in Lösung oder in reiner Form eingesetzt wird. Die Angaben zum Volumen des Katalysators beziehen sich auf das Schüttvolumen.

Die Reaktionstemperatur richtet sich nach der Aktivität des Katalysators, der Menge an Lösungsmittel sowie der gewählten Belastung des Katalysators. Geeignete Reaktionstemperaturen liegen im allgemeinen bei 80 bis 250 °C, bevorzugt 100 bis 180 °C, besonders bevorzugt 110 bis 170 °C.

Das erfindungsgemäße Verfahren wird im allgemeinen bei einem Druck von 5 bis 350 bar, bevorzugt 50 bis 250 bar, durchgeführt, der durch das entsprechende Aufpressen von Wasserstoff eingestellt wird.

Nach Durchgang durch den Reaktor wird bevorzugt ein Teil des Austrags nach dessen Durchgang durch einen Wärmetauscher in den Reaktor zurückgeführt (Flüssigkeitsrückführung), um die Reaktionswärme abzuführen. Die zurückgeführte Menge pro Zeiteinheit ist abhängig von der Katalysatorbelastung und der Reaktorgröße. Sie beträgt im allgemeinen die 1- bis 20-fache Menge des Zulaufs. Bevorzugt wird die zurückgeführte Menge pro Zeiteinheit möglichst groß eingestellt, d. h. bei maximaler Fördermenge der Rückführpumpe gearbeitet.

Die Aufarbeitung des Reaktionsaustrags und Isolierung des Verfahrensproduktes kann nach den üblichen Methoden, z. B. durch fraktionierte kontinuierliche oder diskontinuierliche Destillation bzw. Rektifikation erfolgen. Die Rektifikation kann beispielsweise bei Normaldruck oder geringem Unter- oder Überdruck, bei Rücklaufverhältnissen von 1:1 bis 10:1 und in Kolonnen mit 5 bis 60 theoretischen Böden erfolgen.

Wird im Verfahren ein Lösungsmittel eingesetzt, so kann (mit Ausnahme von Wasser als Lösungsmittel) dieses bei der Destillation zurückgewonnen und in die Reaktion zurückgeführt werden.

Wird im Verfahren ein molarer Überschuß an Diisopropylamin bezogen auf Acetaldehyd eingesetzt, ist die Wiedergewinnung des Amins und anschließende Rückführung in die Synthese besonders wirtschaftlich.

### Beispiele

### Beispiel 1 :

In einen elektrisch beheizten Rohrreaktor wurden 800 ml eines Katalysators eingebaut, der aus 1 % Pd auf 19 % MgO und 80 % Al₂O₃ in Strangform bestand. Der Katalysator wurde drucklos bei 200 °C reduziert. Bei 100 °C und 200 bar Wasserstoffdruck wurden stündlich 87 g Diisopropylamin und 48 g Acetaldehyd (Molverhältnis Amin/Aldehyd = 0,8) in Sumpffahrweise über den Katalysator geleitet (Belastung: 0,06 kg_{Acetaldehyd}l_{Kat.}⁻¹h⁻¹). Die zurückgeführte Menge betrug 2 1 h⁻¹. Bei quantitativem Acetaldehydumsatz betrug die Selektivität zur Hünig-Base 46,9 Mol-%.

### Beispiel 2:

In einen elektrisch beheizten Rohrreaktor wurden 800 ml eines Katalysators eingebaut, der aus 0,9 % Pd und 0,1 % Pt auf ZrO₂ in Strangform bestand. Der Katalysator wurde drucklos bei 140 °C reduziert. Bei 120 °C und 200 bar Wasserstoffdruck wurden stündlich 90 g Diisopropylamin und 50 g Acetaldehyd (Molverhältnis Amin/Aldehyd = 0,8) in Sumpffahrweise über den Katalysator geleitet (Belastung: 0,06 kg_{Acetaldehyd}l_{Kat.}⁻¹h⁻¹). Die zurückgeführte Menge betrug 3 1 h⁻¹. Bei quantitativem Acetaldehydumsatz betrug die Selektivität zur Hünig-Base 71,3 Mol-%.

Nach 7 Tagen wurde der Zulauf auf 181 g Diisopropylamin und 78 g Acetaldehyd (Molverhältnis Amin/Aldehyd = 1,0) umgestellt (Belastung: 0,10 kg_{Acetaldehyd}l_{Kat.}⁻¹h⁻¹). Bei quantitativem Acetaldehydumsatz betrug die Selektivität zur Hünig-Base 68,3 Mol-%.

Nach 18 Tagen wurde die Zulaufmischung auf stündlich 361 g Diisopropylamin und 100 g Acetaldehyd (Molverhältnis Amin/Aldehyd = 1,6) umgestellt (Belastung: 0,13 kg_{Acetaldehyd}l_{Kat.}⁻¹h⁻¹). Bei quantitativem Acetaldehydumsatz betrug die Selektivität zur Hünig-Base 65,6 Mol-%.

### Beispiel 3:

In einen elektrisch beheizten Rohrreaktor wurden 800 ml eines Katalysators eingebaut, der aus 0,5 % Pd auf α-Al₂O₃ in Strangform bestand. Der Katalysator wurde drucklos bei 140 °C reduziert. Bei 120 °C und 200 bar Wasserstoffdruck wurden stündlich 181 g Diisopropylamin und 50 g Acetaldehyd (Molverhältnis Amin/Aldehyd = 1,6) in Sumpffahrweise über den Katalysator geleitet (Belastung: 0,06 kg_{Acetaldehyd}l_{Kat.}⁻¹h⁻¹). Die zurückgeführte Menge betrug 3 1 h⁻¹. Bei quantitativem Acetaldehydumsatz betrug die Selektivität zur Hünig-Base 62,7 Mol-%.

Nach 4 Tagen wurde der Zulauf auf stündlich 361 g Diisopropylamin und 100 g Acetaldehyd (Molverhältnis Amin/Aldehyd = 1,6) umgestellt (Belastung: 0,13 kg_{Acetaldehyd}l_{Kat.}⁻¹h⁻¹). Bei quantitativem Acetaldehydumsatz betrug die Selektivität zur Hünig-Base 49,7 Mol-%.

### Beispiel 4:

In einen elektrisch beheizten Rohrreaktor wurden 800 ml eines Katalysators eingebaut, der aus 0,3 % Pd, 4,6 % Ag und 1 % Mn₂O₃ auf SiO₂ in Strangform bestand. Der Katalysator wurde bei 200 bar und 140 °C reduziert. Bei 140 °C und 200 bar Wasserstoffdruck wurden stündlich 229,5 g Diisopropylamin, 98 g Acetaldehyd (Molverhältnis Amin/Aldehyd = 1,0) und 153 g Ethanol als Lösungsmittel in Sumpffahrweise über den Katalysator geleitet (Belastung: 0,12 kg_{Acetaldehyd}l_{Kat.}⁻¹h⁻¹). Die zurückgeführte Menge betrug 3 1 h⁻¹: Bei quantitativem Acetaldehydumsatz betrug die Selektivität zur Hünig-Base 55,1 Mol-%.

Nach 26 Tagen wurde der Zulauf auf stündlich 101,1 g Diisopropylamin, 50 g Acetaldehyd (Molverhältnis Amin/Aldehyd = 1,0) und 50 g Wasser als Lösungsmittel umgestellt (Belastung: 0,06 kg_{Acetaldehyd}l_{Kat.}⁻¹h⁻¹). Bei quantitativem Acetaldehydumsatz betrug die Selektivität zur Hünig-Base 45,6 Mol-%.

Nach 33 Tagen wurde der Zulauf auf stündlich 166,1 g Diisopropylamin und 50 g Acetaldehyd (Molverhältnis Amin/Aldehyd = 1,5) umgestellt (Belastung: 0,06 kg_{Acetaldehyd}l_{Kat.}⁻¹h⁻¹). Bei quantitativem Acetaldehydumsatz betrug die Selektivität zur Hünig-Base 46,2 Mol-%.

Nach 37 Tagen wurde der Zulauf auf stündlich 332,1 g Diisopropylamin und 50 g Acetaldehyd (Molverhältnis Amin/Aldehyd = 3,0) umgestellt (Belastung: 0.06 kg_{Acetaldehyd}l_{Kat.}⁻¹h⁻¹). Bei quantitativem Acetaldehydumsatz betrug die Selektivität zur Hünig-Base 49,4 Mol-%.

### Beispiel 5:

In einen elektrisch beheizten Rohrreaktor wurden 800 ml eines Katalysators eingebaut, der aus 0,5 % Pd auf γ-Al₂O₃ in Strangform bestand. Der Katalysator wurde drucklos bei 140 °C reduziert. Bei 120 °C und 200 bar Wasserstoffdruck wurden stündlich 181 g Diisopropylamin und 50 g Acetaldehyd (Molverhältnis Amin/Aldehyd = 1,6) in Sumpffahrweise über den Katalysator geleitet (Belastung: 0,06 kg_{Acetaldehyd}l_{Kat.}⁻¹h⁻¹). Die zurückgeführte Menge betrug 3 1 h⁻¹. Bei quantitativem Acetaldehydumsatz betrug die Selektivität zur Hünig-Base 76,3 Mol-%.

Nach 16 Tagen wurde der Zulauf auf stündlich 361 g Diisopropylamin und 100 g Acetaldehyd (Molverhältnis Amin/Aldehyd = 1,6) umgestellt (Belastung: 0,13 kg_{Acetaldehyd}l_{Kat.}⁻¹h⁻¹) und die Temperatur auf 140 °C erhöht. Bei quantitativem Acetaldehydumsatz betrug die Selektivität zur Hünig-Base 64,5 Mol-%.

### Beispiel 6:

In einen elektrisch beheizten Rohrreaktor wurden 800 ml eines Katalysators eingebaut, der aus 0,4 % Pt und 0,4 % Pd auf ZrO₂ in Tablettenform bestand. Der Katalysator wurde drucklos bei 140 °C reduziert. Bei 130 °C und 200 bar Wasserstoffdruck wurden stündlich 86,6 g Diisopropylamin und 48 g Acetaldehyd (Molverhältnis Amin/Aldehyd = 0,8) in Sumpffahrweise über den Katalysator geleitet (Belastung: 0,06 kg_{Acetaldehyd}l_{Kat}.⁻¹h⁻¹). Die zurückgeführte Menge betrug 6 1 h⁻¹. Bei quantitativem Acetaldehydumsatz betrug die Selektivität zur Hünig-Base 66,7 Mol-%.

### Beispiel 7:

Analog zu Beispiel 6 wurden bei 120 °C stündlich 216,6 g Diisopropylamin und 96 g Acetaldehyd (Molverhältnis Amin/Aldehyd = 1,0) in Sumpffahrweise über den Katalysator geleitet (Belastung: 0,12 kg_{Acetaldehyd}l_{Kat.}⁻¹h⁻¹). Die zurückgeführte Menge betrug 3 1 h⁻¹. Bei quantitativem Acetaldehydumsatz betrug die Selektivität zur Hünig-Base 53,5 Mol-%.

Nach 42 Tagen wurde die Temperatur auf 130 °C erhöht und der Zulauf auf stündlich 222 g Diisopropylamin, 98 g Acetaldehyd (Molverhältnis Amin/Aldehyd = 1,0) und 55,5 g Ethanol als Lösungsmittel umgestellt (Belastung: 0,12 kg_{Acetaldehyd}l_{Kat.}⁻¹h⁻¹). Bei quantitativem Acetaldehydumsatz betrug die Selektivität zur Hünig-Base 79,9 Mol-%.

Nach 68 Tagen wurde der Zulauf auf stündlich 229,5 g Diisopropylamin, 98 g Acetaldehyd (Molverhältnis Amin/Aldehyd = 1,0) und 153 g Ethanol als Lösungsmittel umgestellt (Belastung: 0,12 kg_{Acetaldehyd}l_{Kat.}⁻¹h⁻¹). Bei quantitativem Acetaldehydumsatz betrug die Selektivität zur Hünig-Base 81,3 Mol-%.

Nach 82 Tagen wurde der Zulauf auf stündlich 286,9 g Diisopropylamin, 123 g Acetaldehyd (Molverhältnis Amin/Aldehyd = 1,0) und 191,2 g Ethanol als Lösungsmittel umgestellt (Belastung: 0,15 kg_{Acetaldehyd}l_{Kat.}⁻¹h⁻¹). Bei quantitativem Acetaldehydumsatz betrug die Selektivität zur Hünig-Base 77,3 Mol-%.

### Beispiel 8:

Analog zu Beispiel 6 wurden bei 120 °C stündlich 180,5 g Diisopropylamin und 50 g Acetaldehyd (Molverhältnis Amin/Aldehyd = 1,6) in Sumpffahrweise über den Katalysator geleitet (Belastung: 0,06 kg_{Acetaldehyd}l_{Kat.}⁻¹h⁻¹). Die zurückgeführte Menge betrug 3 l h⁻¹. Bei quantitativem Acetaldehydumsatz betrug die Selektivität zur Hünig-Base 71,7 Mol-%.

Nach 5 Tagen wurde der Zulauf auf stündlich 270,8 g Diisopropylamin und 75 g Acetaldehyd (Molverhältnis Amin/Aldehyd = 1,6) umgestellt (Belastung: 0,09 kg_{Acetaldehyd}l_{Kat}.⁻¹h⁻¹). Bei quantitativem Acetaldehydumsatz betrug die Selektivität zur Hünig-Base 72,5 Mol-%.

Nach 8 Tagen wurde der Zulauf auf stündlich 361 g Diisopropylamin und 100 g Acetaldehyd (Molverhältnis Amin/Aldehyd = 1,6) umgestellt (Belastung: 0,13 kg_{Acetaldehyd}l_{Kat}.⁻¹h⁻¹). Bei quantitativem Acetaldehydumsatz betrug die Selektivität zur Hünig-Base 71,1 Mol-%.

Nach 12 Tagen wurde der Zulauf auf stündlich 361 g Diisopropylamin und 50 g Acetaldehyd (Molverhältnis Amin/Aldehyd = 3,1) umgestellt (Belastung: 0,06 kg_{Acetaldehyd}l_{Kat.}⁻¹h⁻¹). Bei quantitativem Acetaldehydumsatz betrug die Selektivität zur Hünig-Base 90,0 Mol-%.

Nach 14 Tagen wurde der Zulauf auf stündlich 722 g Diisopropylamin und 100 g Acetaldehyd (Molverhältnis Amin/Aldehyd = 3,0) umgestellt (Belastung: 0,13 kg_{Acetaldehyd}l_{Kat.}⁻¹h⁻¹). Bei quantitativem Acetaldehydumsatz betrug die Selektivität zur Hünig-Base 74,6 Mol-%.

Nach 15 Tagen wurde der Zulauf auf stündlich 541.5 g Diisopropylamin und 150 g Acetaldehyd (Molverhältnis Amin/Aldehyd = 1,6) umgestellt (Belastung: 0,19 kg_{Acetaldehyd}l_{Kat.}⁻¹h⁻¹). Bei quantitativem Acetaldehydumsatz betrug die Selektivität zur Hünig-Base 66,1 Mol-%.

### Beispiel 9:

In einen elektrisch beheizten Rohrreaktor wurden 800 ml eines Katalysators eingebaut, der aus 0,5 % Ru auf γ-Al₂O₃ in Strangform bestand. Der Katalysator wurde drucklos bei 150 °C reduziert. Bei 100 °C und 200 bar Wasserstoffdruck wurden stündlich 181 g Diisopropylamin und 50 g Acetaldehyd (Molverhältnis Amin/Aldehyd = 1,6) in Sumpffahrweise über den Katalysator geleitet (Belastung: 0,06 kg_{Acetaldehyd}l_{Kat.}⁻¹h⁻¹). Die zurückgeführte Menge betrug 3 1 h⁻¹. Bei quantitativem Acetaldehydumsatz betrug die Selektivität zur Hünig-Base 41,1 Mol-%.

### Beispiel 10:

Analog zu Beispiel 6 wurden bei 120 °C und 200 bar Wasserstoffdruck stündlich 361 g Diisopropylamin und 100 g Acetaldehyd (Molverhältnis Amin/Aldehyd = 1,6) in Rieselfahrweise über den Katalysator geleitet (Belastung: 0,13 kg_{Acetaldehyd}l_{Kat.}⁻¹h⁻¹). Die zurückgeführte Menge betrug 6 1 h⁻¹. Bei quantitativem Acetaldehydumsatz betrug die Selektivität zur Hünig-Base 83,3 Mol-%.

Nach 12 Tagen wurde der Zulauf auf stündlich 361 g Diisopropylamin und 50 g Acetaldehyd (Molverhältnis Amin/Aldehyd = 3,1; Katalysatorbelastung: 0,06 kg_{Acetaldehyd}l_{Kat}.⁻¹h⁻¹) umgestellt. Gleichzeitig wurde die Temperatur auf 110 °C abgesenkt. Bei quantitativem Acetaldehydumsatz betrug die Selektivität zur Hünig-Base 87 Mol-%.

### Beispiel 11:

In einen elektrisch beheizten Rohrreaktor wurden 800 ml eines Katalysators eingebaut, der aus 0,5 % Pd auf ZrO₂ in Tablettenform bestand. Der Katalysator wurde drucklos bei 140 °C reduziert. Bei 160 °C und 200 bar Wasserstoffdruck wurden stündlich 180,5 g Diisopropylamin und 50 g Acetaldehyd (Molverhältnis Amin/Aldehyd = 1,6) in Sumpffahrweise über den Katalysator geleitet (Belastung: 0,06 kg_{Acetaldehyd}l_{Kat.}⁻¹h⁻¹). Die zurückgeführte Menge betrug 6 l h⁻¹. Bei quantitativem Acetaldehydumsatz betrug die Selektivität zur Hünig-Base 83,7 Mol-%.

## Patentansprüche

1. Verfahren zur Herstellung von N-Ethyl-diisopropylamin durch Umsetzung von Acetaldehyd mit Diisopropylamin und Wasserstoff bei erhöhter Temperatur und unter Druck in Gegenwart eines Hydrierkatalysators, **dadurch gekennzeichnet, dass** der Katalysator ein oxidisches Trägermaterial, ausgewählt aus der Gruppe Zirkoniumdioxid, Titandioxid, Aluminiumoxid, Siliziumdioxid, Zinkoxid, Magnesiumoxid, Cerdioxid, Tone und Zeolithe oder Mischungen daraus, enthält.

2. Verfahren nach Anspruch 1, **dadurch gekennzeichnet, dass** die katalytisch aktive Masse des Katalysators 50 bis 99,9 Gew.-% ZrO₂, TiO₂, Al₂O₃, SiO₂ und/oder MgO und 0,1 bis 50 Gew.-% Cr, Mo, W, Re, Ru, Rh, Pd, Os, Ir, Pt, Ag, Au, Fe, Co, Ni und/ oder Cu, berechnet als Metall in der Oxidationsstufe 0, enthält.

3. Verfahren nach den Ansprüchen 1 und 2, **dadurch gekennzeichnet, dass** die katalytisch aktive Masse des Katalysators 60 bis 99,9 Gew.-% ZrO₂ enthält.

4. Verfahren nach den Ansprüchen 1 bis 3, **dadurch gekennzeichnet, dass** die Umsetzung in Gegenwart von überschüssigem Diisopropylamin durchgeführt wird.

5. Verfahren nach den Ansprüchen 1 bis 4, **dadurch gekennzeichnet, dass** unumgesetztes Diisopropylamin wieder in die Synthese zurückgeführt wird.

6. Verfahren nach den Ansprüchen 1 bis 5, **dadurch gekennzeichnet, dass** die Umsetzung kontinuierlich in einem Festbettreaktor mit Flüssigkeitsrückführung durchgeführt wird.

7. Verfahren nach den Ansprüchen 1 bis 6, **dadurch gekennzeichnet, dass** die Umsetzung in Gegenwart eines inerten Lösungsmittels durchgeführt wird.

8. Verfahren nach den Ansprüchen 1 bis 7, **dadurch gekennzeichnet, dass** die Umsetzung bei Temperaturen von 80 bis 250 °C durchgeführt wird.

9. Verfahren nach den Ansprüchen 1 bis 8, **dadurch gekennzeichnet, dass** die Umsetzung bei Drücken von 5 bis 350 bar durchgeführt wird.

## Claims

1. A process for preparing N-ethyldiisopropylamine by reacting acetaldehyde with diisopropylamine and hydrogen at elevated temperature and under pressure in the presence of a hydrogenation catalyst which comprises the catalyst comprising an oxidic support material selected from the group consisting of zirconium dioxide, titanium dioxide, aluminum oxide, silicon dioxide, zinc oxide, magnesium oxide, cerium dioxide, clays and zeolites or mixtures thereof.

2. A process as claimed in claim 1, wherein the catalytically active mass of the catalyst comprises from 50 to 99.9% by weight of ZrO₂, TiO₂, Al₂O₃, SiO₂ and/or MgO and from 0.1 to 50% by weight of Cr, Mo, W, Re, Ru, Rh, Pd, Os, Ir, Pt, Ag, Au, Fe, Co, Ni and/or Cu, calculated as metals in the oxidation state 0.

3. A process as claimed in claim 1 or 2, wherein the catalytically active mass of the catalyst comprises from 60 to 99.9% by weight of ZrO₂.

4. A process as claimed in any of claims 1 to 3, wherein the reaction is carried out in the presence of excess diisopropylamine.

5. A process as claimed in any of claims 1 to 4, wherein unreacted diisopropylamine is recycled back to the synthesis.

6. A process as claimed in any of claims 1 to 5, wherein the reaction is carried out continuously in a fixed-bed reactor with liquid recirculation.

7. A process as claimed in any of claims 1 to 6, wherein the reaction is carried out in the presence of an inert solvent.

8. A process as claimed in any of claims 1 to 7, wherein the reaction is carried out at from 80 to 250°C.

9. A process as claimed in any of claims 1 to 8, wherein the reaction is carried out at pressures from 5 to 350 bar.

## Revendications

1. Procédé de fabrication de N-éthyl-di-isopropylamine par réaction de l'acétaldéhyde avec le di-isopropylamine et l'hydrogène, à une température élevée et sous pression, en présence d'un catalyseur d'hydrogénation, **caractérisé en ce que** le catalyseur contient une matière support de type oxyde choisi dans le groupe formé par le dioxyde de zirconium, le dioxyde de titane, l'oxyde d'aluminium, le dioxyde de silicium, l'oxyde de zinc, l'oxyde de magnésium, le dioxyde de cérium, les argiles et les zéolithes ou leurs mélanges

2. Procédé selon la revendication 1, **caractérisé en ce que** la masse catalytique active du catalyseur contient 50% à 99,9% en poids de ZrO₂, TiO₂, Al₂O₃, SiO₂ et/ou MgO, et 0,1% à 50% en poids de Cr, Mo, W, Re, Ru, Rh, Pd, Os, Ir, Pt, Ag, Au, Fe, Co, Ni et/ou Cu, exprimé en métal ayant un degré d'oxydation 0.

3. Procédé selon les revendications 1 et 2, **caractérisé en ce que** la masse catalytique active du catalyseur contient 60% à 99,9% en poids de ZrO₂.

4. Procédé selon les revendications 1 à 3, **caractérisé en ce que** l'on réalise la réaction en présence de di-isopropylamine en excès.

5. Procédé selon les revendications 1 à 4, **caractérisé en ce que** l'on recycle dans l'étape de synthèse le di-isopropylamine qui n'a pas réagi.

6. Procédé selon les revendications 1 à 5, **caractérisé en ce que** l'on réalise la réaction en continu, dans un réacteur à lit fixe, en recyclant le liquide.

7. Procédé selon les revendications 1 à 6, **caractérisé en ce que** l'on réalise la réaction en présence d'un solvant inerte.

8. Procédé selon les revendications 1 à 7, **caractérisé en ce que** l'on réalise la réaction à une température comprise entre 80°C et 250°C.

9. Procédé selon les revendications 1 à 8, **caractérisé en ce que** l'on réalise la réaction à une pression comprise entre 5 bars et 350 bars.
